# EUROPEAN PATENT APPLICATION

(11) **EP 2 659 834 A1**
(43) Date of publication of application: **06.11.2013**
(21) Application number: 12166136.7
(22) Date of filing: 30.04.2012
(51) Int. Cl.: A61B 5/07, A61B 5/1473, A61B 5/145

(54) **Costoptimized Hp capsule**

(71) Applicant: Siemens Aktiengesellschaft, 80333 München (DE)
(72) Inventor: Förtsch, Stefan, 91358 Kunreuth (DE); Kuth, Rainer, 91315 Höchstadt (DE)

(57) **Abstract**

The invention relates to a method for producing an ingestible capsule, comprising at least two electrodes 3 affixed to a substrate 1 being embedded in a resin material 13, wherein at least a portion of each electrode 3 is exposed. The capsule can be used to detect Helicobacter pylori (Hp).

## Description

The invention relates to a method for producing an ingestible capsule and the capsule produced by said method.

A frequent cause for complaints of the upper gastrointestinal tract is a bacterial infection of its organs. For example, an infection by Helicobacter pylori has been held responsible for a large range of stomach illnesses that are accompanied by an intensified secretion of stomach acid. Type B gastritis, approximately 75% of gastric ulcers and nearly all duodenal ulcers fall into this category, for example. The examination of the hollow organs of the gastrointestinal tract for colonization by bacteria-in particular for colonization with Helicobacter pylori-is therefore an important component of the diagnosis of stomach illnesses.

EP2265168 describes an endocapsule for detecting the presence of the bacterium in the gastrointestinal tract by means of at least one of its metabolic products. The endocapsule is introduced into the gastrointestinal tract and that contains a sensor with electrodes for detecting the metabolic product.

EP2398374 describes an endoscopic capsule comprising a biocompatible housing which houses at least one sensor device arranged on the outer surface of the housing, the sensor having a first electrode and a second electrode for the detection of a metabolic product of Helicobacter pylori.

For this type of application it is desirable to use a single use capsule. A single use capsule for detecting Helicobacter Pylori or other diseases requiring outer electrodes with connections to inside electronics should have an architecture which is very effective for manufacturing.

Current capsules for swallowing do not have outer electrodes with connections to an inside electronic. E.g. the very first cardiac pacemaker was a simple electronic circuit in a resin housing.

It is therefore an object of the present invention to provide a cost effective method for producing an ingestible capsule having a sensor.

### Summary of the invention

The invention relates to a method for producing an ingestible capsule and the capsule produced by said method. As used herein, "ingestible" means that the capsule can be swallowed by a human or animal patient. Preferably, the capsules can be designed in a way that it can pass the gastrointestinal tract without injuring the patient.

In general terms, the invention relates to a method for producing an ingestible capsule, comprising at least two electrodes affixed to a substrate being embedded in a resin material, wherein at least a portion of each electrode is exposed. An exemplary use of such a capsule is for the detection of Helicobacter pylori (Hp).

The method according to the invention comprises the following steps
a) affixing two electrodes to a substrate
b) embedding said substrate with the affixed electrodes in a resin material, wherein at least a portion of each electrode is exposed.

The term "exposed" means that the exposed portion of an electrode is not covered by the resin material, i.e. it is accessible to the outside environment for sensing.

According to an aspect of the invention the method comprises embedding the substrate in resin material so that at least a portion of the electrodes remains exposed. This can e.g. be achieved by ensuring that not the entire electrode is covered by resin material when the substrate is embedded in the resin material.

According to an alternative aspect of the invention the method comprises removing resin material so that at least a portion of the electrodes is exposed. This can e.g. be achieved by milling, etching or any other suitable process for selectively removing resin material.

According to an aspect of the invention the step of embedding the substrate in a resin material comprises placing said substrate with the affixed electrodes in a casting mold and filling said casting mold with a resin material.

The resin material may be a thermoplastic resin material which can be solidified by cooling down.

The resin material may be a curable resin material which can be solidified by curing.

According to an aspect of the invention the substrate comprises a printed circuit board.

According to an aspect of the invention the electrodes are affixed to the substrate by soldering.

According to an aspect of the invention additional electronic components and, optionally, a battery are provided. Preferably they are also affixed to the circuit board.

According to an aspect of the invention the exposed portion of the electrodes are located in a recessed portion of the capsule. Optionally this recessed portion of the capsule can be covered with a membrane that is permeable, e.g. to a metabolic product of an organism which should be detected. An example for this would be Helicobacter pylori which produces ammonia as a metabolic product. In this case the membrane should be ammonia-permeable.

According to an aspect of the invention the capsule comprises an activation mechanism.

According to an aspect of the invention the activation mechanism is selected from the group consisting of a reed-contact and an activation mechanism comprising a light detector.

According to an aspect of the invention multiple substrates for multiple capsules are provided as a joint substrate, comprising the step of separating the joint substrate into single substrates.

According to an aspect of the invention the electrodes and, optionally, other electronic components are SMD-devices (surface mount devices), and the method comprises placing on the substrate by a placing robot.

According to an aspect of the invention the capsule comprises an indication light which is activated when the capsule is active or transmitting data. The indication lights can indicate that the capsule is activated and functioning properly. An LED can be used as indication light.

The invention further relates to an ingestible capsule, comprising two electrodes affixed to a substrate, said substrate being embedded in a resin material, wherein at least a portion of each electrode is exposed, i.e. not covered by the resin material.

According to an aspect of the invention the capsule comprises a radio-frequency identification (RFID) tag.

According to an aspect of the invention a first electrode of the two electrodes is made of a noble metal, which cannot be attacked by acid, and a second electrode of the two electrodes is made of silver.

The noble metal can e.g. be gold or platinum.

According to an aspect of the invention the second electrode has a silver chloride layer.

### Detailed description of the invention

Before the invention is described in detail, it is to be understood that this invention is not limited to the particular component parts of the process steps of the methods described as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

The invention will be described in connection to examples and figures (images) which show in
Fig. 1: a schematic view of a PCB which can be used in the invention;
Fig. 2: a top view of a PCB which can be used in the invention;
Fig. 3: a schematic view of the process of affixing an electrode to a PCB;
Fig. 4: a schematic view of the process of embedding the PCB in a resin material; and
Fig. 5: a schematic view of the process of manufacturing individual substrates from a larger multiple substrate

The entire skeleton of the ingestible capsule comprises a substrate, e.g. a PCB (printed circuit board).

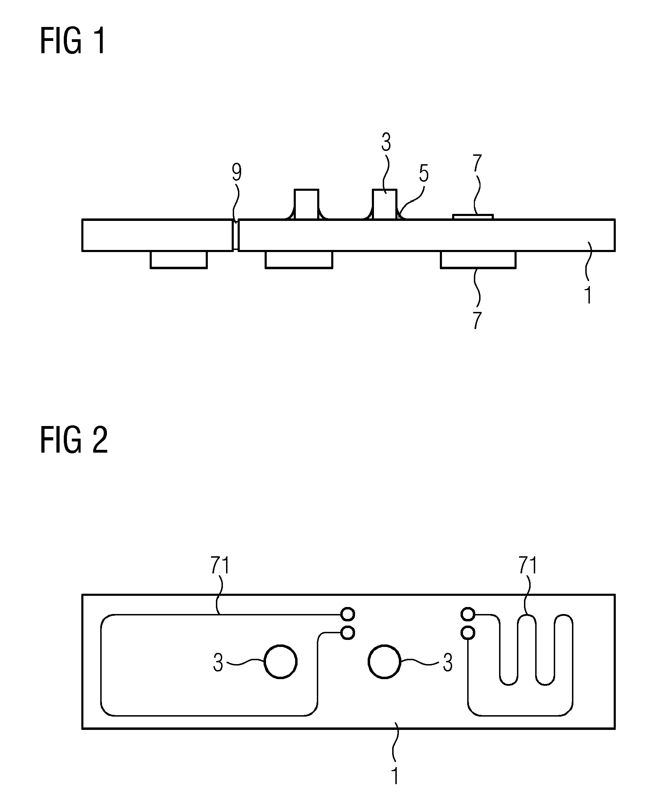

In Fig. 1 the substrate 1 is a printed circuit board (PCB) which supports the electrodes 3 and other electronic components 7. If electronic components are arranged on both sides of the PCB, an interlayer connection 9 is provided.

The two electrodes 3 are affixed to the substrate, e.g. they are soldered on the PCB with soldering connections 5. The electrodes 3 can be massive (of electrode surface material) or coated , e.g. coated cylinders, domes or rectangular solids.

The other electronic components 7 can be affixed, e.g. by soldering on the same and/or the opposite side of the substrate 1.

Advantageously any components on the same side as the electrodes 3 should have a flatter profile than the electrodes.

Preferably, a battery should be soldered on the PCB as well, if a battery is required.

A battery is not required if the electronic is configured as RFID tag.

Fig. 2 shows a top view of a substrate wherein antennas 71 are provided and arranged in a flat pattern, e.g. a meandering pattern on the PCB 1. Such an antenna, e.g. an radio frequency (rf) antenna, can be partly or completely a printed wiring.

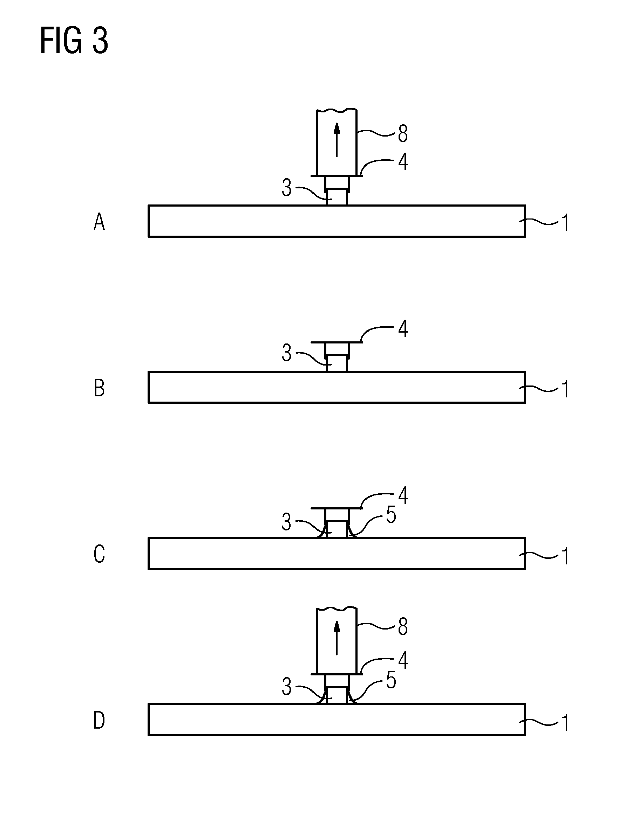

Preferably the electrodes 3 and -optionally - also the other electronic components 7 are designed as SMD-devices (surface mount devices) so that a placing robot can place them on the substrate, e.g. on the PCB prior to soldering. This requires a minimal horizontal surface which can e.g.be supplied by a removable cover 4 (Fig. 3).

Fig. 3 shows a schematic view of the process of affixing an electrode 3 to a PCB 1 with a placement robot 8. The electrodes 3 can optionally have a cap 4 which is removed after soldering to allow a placement robot 8 to place electrodes. The placement robot has a vacuum arm 8.
A: During placing a vacuum is applied to a plastic cap 4. The cap is removably attached to an electrode 3.
B: The electrode 3 is placed on the board 1.
C: A soldering connection 5 is made to affix the electrode 3 to the PCB 1.
D: After soldering the vacuum arm 8 can remove the plastic cap 3 from the affixed electrode 1.

Optionally, the electrode has an additional SMD led which is activated when the capsule is active or transmitting data.

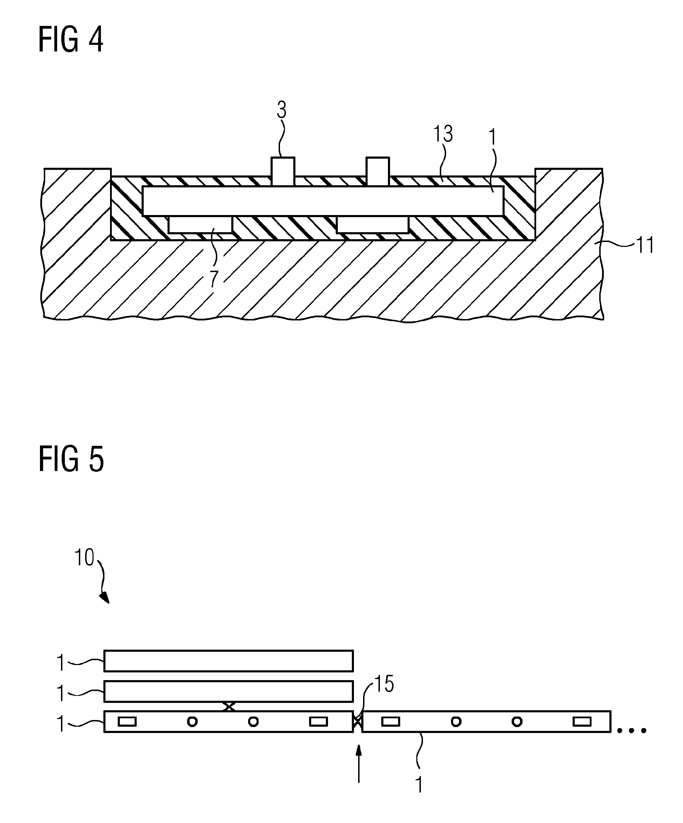

After all electronic components are attached to the PCB substrate 1 is placed in a casting mold 11 (Fig. 4). This mold is filled with a resin 13 or other plastic material, so that the electrodes 3 are not covered completely. Alternatively the electrodes could be covered at first and then exposed by removal of resin material, e.g. by additional etching or milling after the resin is solidified. As resin material thermoplastic resins, curable resins and curable elastomeric resins can be used. The resin material should be biocompatible, i.e. inert and non-toxic. A non-limiting list of examples includes polyolfines, silicone materials, polytetrafluorethylene, polyvinylfluoride and others, as are known in the art.

The capsule advantageously has a shape that facilitates ingestion by swallowing, e.g. a rounded shape such as an elliptical or oval shape.

The capsule can include an activation mechanism. This can be a reed-contact: e.g. in off mode the capsule is in close proximity to an external permanent magnet. Alternatively the capsule could have a light detector: if bright for more than e.g. 10s the capsule is activated. This would require a light-shielded packaging. Alternatively the capsule could be activated by a pulsed light.

Multiple substrates 1 for multiple capsules are manufactured as one big substrate 10, which is cut into single substrates for each capsule at the end of the manufacturing process. The PCB material can be etched or milled until only a small, breakable connection 15 remains (Fig. 5).

Helicobacter pylori (Hp) produces ammonia (NH₃) as a metabolic product. As an example, for the detection of Helicobacter pylori, the first electrode of the two electrodes is made of a noble metal, which cannot be attacked by acid, and a second electrode of the two electrodes is made of silver. The noble metal can e.g. be gold or platinum. The second electrode is a silver electrode with a silver chloride layer. An electric voltage can be applied between the first electrode and the second electrode, and a change in an electric variable can be measured if ammonia is present between the first electrode and the second electrode. This is due to ammonia reacting with silver chloride to form a silverdiamine complex, Ag(NH₃)₂⁺. Thereby the AgCl layer on the silver electrode is dissolved and a resulting change in electric properties at the electrode can be detected.

After a patient has swallowed the capsule, the sensor detects ammonia (NH₃) present in the gastric acid and in the tissue of the mucosa of the stomach on the stomach inner wall. This is used to detect affliction of the tissue (mucosa of the stomach) with Helicobacter pylori in a patient-friendly fashion by detecting ammonia (NH3). This takes place without a biopsy and therefore puts much less strain on the patient.

The detection of ammonia is a very strong indication for the presence of Helicobacter pylori because ammonia is generated by the Helicobacter pylori bacteria by splitting urea using urease in order to protect itself from the acidic environment of the gastrointestinal tract, more particularly the high acid concentration in the stomach.

The method and capsule of the invention provide a very cost effective way to manufacture ingestible capsules, e.g. for detection of Helicobacter pylori.

## Claims

1. Method for producing an ingestible capsule, comprising:
a) affixing at least two electrodes to a substrate and
b) embedding said substrate with the affixed electrodes in a resin material,
wherein at least a portion of the electrodes are exposed so that they are not covered by the resin material.

2. Method according to claim 1, comprising embedding the substrate in resin material so that at least a portion of the electrodes remains exposed.

3. Method according to claim 1, comprising removing resin material so that at least a portion of the electrodes is exposed.

4. Method according to any of the aforementioned claims, wherein the substrate comprises a printed circuit board.

5. Method according to any of the aforementioned claims, wherein the electrodes are affixed to the substrate by soldering.

6. Method according to any of the aforementioned claims, wherein additional electronic components and, optionally, a battery are affixed to the substrate.

7. Method according to any of the aforementioned claims, wherein the capsule comprises an RFID tag.

8. Method according to any of the aforementioned claims, wherein the capsule comprises an activation mechanism.

9. Method according to claim 8, wherein the activation mechanism is selected from the group consisting of a reed-contact and an activation mechanism comprising a light detector.

10. Method according to any of the aforementioned claims, wherein multiple substrates for multiple capsules are provided as a joint substrate, comprising the step of separating the joint substrate into single substrates.

11. Method according to any of the aforementioned claims, wherein the electrodes and, optionally, other electronic components are SMD-devices (surface mount devices), comprising placing on the substrate by a placing robot.

12. Method according to any of the aforementioned claims, wherein the capsule comprises a indication light which is activated when the capsule is active or transmitting data.

13. An ingestible capsule, comprising at least two electrodes affixed to a substrate being embedded in a resin material, wherein at least a portion of each electrode is exposed.

14. The capsule according to claim 13, wherein the substrate comprises a printed circuit board.

15. The capsule according to claim 13 or 14, wherein the capsule comprises a battery.

16. The capsule according to claim 13 or 14, wherein the capsule comprises an RFID tag.

17. The capsule according to any of claims 13 to 16, wherein the capsule comprises an activation mechanism.

18. The capsule according to claim 17, wherein the activation mechanism is selected from the group consisting of a reed-contact and an activation mechanism comprising a light detector.

19. The capsule according to any of claims 13 to 18, wherein the capsule comprises an indication light which is activated when the capsule is active or transmitting data.

20. The capsule according to any of claims 13 to 18, wherein a first electrode of the two electrodes is made of a noble metal, which cannot be attacked by acid, and a second electrode of the two electrodes is made of silver.
